# EUROPÄISCHE PATENTSCHRIFT

(11) **EP 1 311 492 B1**
(45) Veröffentlichungstag und Bekanntmachung des Hinweises auf die Patenterteilung: **08.09.2004**
(21) Anmeldenummer: 02758308.7
(22) Anmeldetag: 04.07.2002
(51) Int. Cl.: C07D 253/06, C07C 281/18, C07C 257/14, C07C 255/61, C07C 251/30

(54) **VERFAHREN ZUR HERSTELLUNG VON LAMOTRIGIN AUS ALPHA-OXO-2,3-DICHLORPHENYLACETAMIDINO-AMINOGUANIDINO-HYDRAZON DURCH RINGSCHLUSSREAKTION**
METHOD FOR PRODUCING LAMOTRIGINE FROM ALPHA-OXO-2,3-DICHLOROPHENYL ACETAMIDINO-AMINOGUANIDINO HYDRAZONE BY RING CLOSURE REACTION
PROCEDE DE FABRICATION DE LAMOTRIGINE A PARTIR DE ALPHA-OXO-2,3-DICHLORPHENYLACETAMIDINO-AMINOGUANIDINO-HYDRAZONE PAR REACTION AVEC FERMETURE DU CYCLE

(30) Priorität: 17.07.2001 DE 10134980
(43) Veröffentlichungstag der Anmeldung: 21.05.2003
(73) Patentinhaber: Helm AG, 20097 Hamburg (DE); CF Pharma Gyogyszergyarto Kft., 1097 Budapest (HU)
(72) Erfinder: SCHNEIDER, Géza, H-1097 Budapest, Ungarn (HU); GEGÖ, Csaba, Lehel, H-1181 Budapest (HU); ONDI, Levente, H-1078 Budapest (HU); MATE, Attila, Gergely, H-1188 Budapest (HU); LUKACS, Ferenc, H-2143 Kistarcsa, Ungarn (HU); NYERGES, Miklús, H-1122 Budapest, Ungarn (HU); GARACZI, Sándor, H-1145 Budapest, Ungarn (HU)
(74) Vertreter: Teipel, Stephan, Dr.
(86) Internationale Anmeldenummer: PCT/EP2002/007433
(87) Internationale Veröffentlichungsnummer: WO 2003/008393

(56) Entgegenhaltungen:
- EP-A- 0 963 980
- WO-A-00/35888
- WO-A-01/49669
- WO-A-96/20934

## Beschreibung

Die vorliegende Erfindung betrifft ein Verfahren zur Herstellung von Lamotrigin sowie in diesem Verfahren eingesetzte Zwischenprodukte.

Lamotrigin (3,5-Diamino-6-(2,3-dichforphenyl)-1,2,4-triazin) hat die Formel I

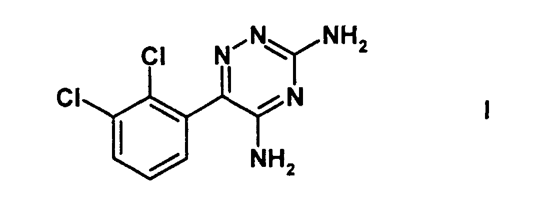

Diese beispielsweise in der EP-A-0 021 121 offenbarte Verbindung eignet sich zur Behandlung von Erkrankungen des Zentralnervensystems, insbesondere von Epilepsie, wobei sie in krampflösenden Arzneimitteln seit 1990 in zahlreichen Ländern gebräuchlich ist.

Es wurden bislang verschiedene Verfahren zur Herstellung von Lamotrigin offenbart. Den in den EP-A-0 021 121, EP-A-0 247 892, EP-A-0 963 980 und WO 00/35888 offenbarten Verfahren ist gemein, daß der Ringschluß von 2-(2,3-Dichlorphenyl)-2-(guanidinylimino)acetonitril (Formel II) der Schlußschritt der Synthese ist.

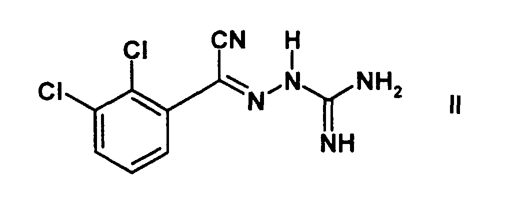

Die für die Ringschlußreaktion benötigte Verbindung der Formel II kann auf verschiedenen Wegen erhalten werden. Das nachfolgende Reaktionsschema veranschaulicht die in der EP-A-0 963 980 offenbarte Reaktionsfolge:

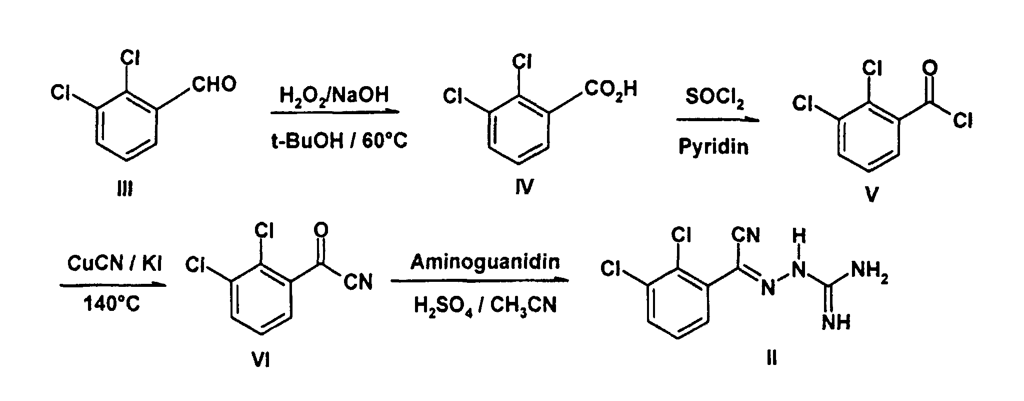

Die in den vorstehend genannten Patentanmeldungen offenbarten Verfahren zur Herstellung von Lamotrigin über eine Ringschlußreaktion mit einer Verbindung der Formel II weichen in einzelnen Schritten von der vorstehend dargestellten Reaktionsfolge ab. Gemeinsam ist ihnen jedoch, daß die Verbindung der Formel II durch die Umsetzung von 2,3-Dichlorbenzoylcyanid (Formel VI) mit Aminoguanidin erhalten wird. Das 2,3-Dichlorbenzoylcyanid ist gemäß diesen Patentanmeldungen durch Umsetzung mit Kupfercyanid aus 2,3-Dichlorbenzoylchlorid (Formel V) zugänglich.

Der vorstehend beschriebene Syntheseweg zur Herstellung von Lamotrigin hat den Nachteil, daß das bei der Synthese der Verbindung der Formel II benötigte 2,3-Dichlorbenzoylcyanid (Formel Vl) nur in kaum zu reinigender, öliger Form erhalten wird und diese Verbindung zudem instabil ist und zu Dimerisierung neigt. Außerdem ist das für die Synthese des 2,3-Dichlorbenzoylcyanids (Formel VI) benötigte Kupfercyanid relativ teuer.

Darüber hinaus fällt bei der Synthese des 2,3-Dichlorbenzoylcyanids (Formel VI) 2,3-Dichlorbenzoesäureanhydrid als Verunreinigung an. Dieses Anhydrid kann gemäß der EP-A-0 963 980 in der weiteren Reaktionsfolge mit Lamotrigin zu einer Verbindung der Formel VII 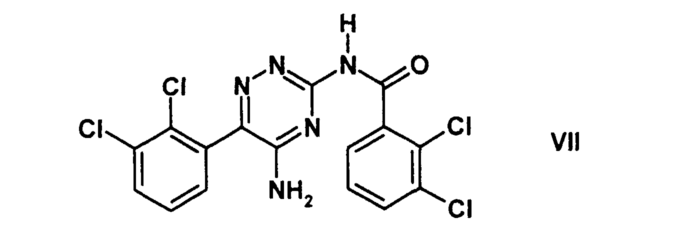 reagieren, die eine unerwünschte Verunreinigung darstellt. Als zusätzliche unerwünschte Verunreinigung nennt die EP-A-0 963 980 eine Verbindung der Formel VIII 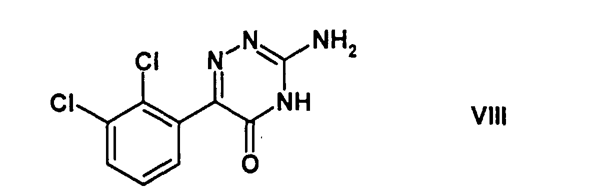 die durch Hydrolyse von Lamotrigin unter alkalischen Bedingungen entsteht. Daher sind einige der in den vorstehend genannten Patentanmeldungen offenbarten Synthesewege zur Herstellung von Lamotrigin auch deshalb nachteilig, da die Ringschlußreaktion der Verbindung der Formel 11 in stark alkalischer Umgebung durchgeführt wird, so daß bereits während der Synthese des Lamotrigins durch Hydrolyse die unerwünschte Verunreinigung VIII entstehen kann.

Eine alternative Synthese für Lamotrigin wird in der WO 96/20934 offenbart. Hier wird die Ringschlußreaktion mit einer zu der Verbindung der Formel II analogen Verbindung durchgeführt, die an Stelle der Cyanidgruppe eine Säureamidgruppe enthält. Diese Ringschlußreaktion muß photochemisch angeregt werden. Dies ist technisch jedoch immer mit Problemen verbunden.

Die WO 96/20935 offenbart ein Verfahren zur Herstellung von Lamotrigin aus 6-(2,3-Dichlorphenyl)-3-methylthio-5-chlor-1,2,4-triazin. Beim Nacharbeiten dieses Verfahrens konnten in der Reaktionsmischung mittels HPLC neben Lamotrigin auch größere Menge unbekannter Nebenprodukte nachgewiesen werden.

Es besteht somit weiterhin ein Bedürfnis, nach Verfahren mit denen Lamotrigin industriell möglichst wirtschaftlich und in möglichst großer Reinheit erhalten werden kann.

Eine Aufgabe der vorliegenden Erfindung bestand somit darin, ein Verfahren zur Herstellung von Lamotrigin zur Verfügung zu stellen, das die vorstehend genannten Nachteile überwindet.

Es wurde nun überraschend gefunden, daß Lamotrigin mittels Ringschlußreaktion in hoher Reinheit aus einer bislang nicht beschriebenen Zwischenverbindung erhalten werden kann.

Die vorliegende Erfindung betrifft somit ein Verfahren zur Herstellung von Lamotrigin oder eines pharmazeutisch verträglichen Salzes davon, worin mit einer Verbindung der Formel XII 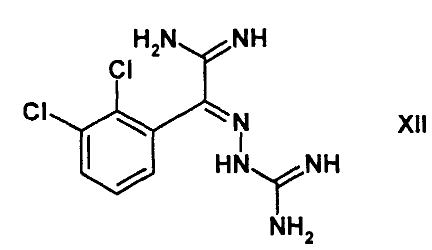 oder einem Salz davon eine Ringschlußreaktion durchgeführt wird und gegebenenfalls aus dem dabei erhaltenen Salz der Verbindung der Formel I die freie Base freigesetzt wird und, falls gewünscht, diese in ein pharmazeutisch verträgliches Salz überführt wird.

Durch die erfindungsgemäße Ringschlußreaktion mit der Verbindung XII zu Lamotrigin wird dieses unter milden Bedingungen in hoher Ausbeute und besonders hoher Reinheit erhalten. Hinzu kommt, daß die Verbindung der Formel XII über einen einfachen Syntheseweg leicht zugänglich ist und durch Kristallisation gut zu reinigen ist. Durch die gegenüber den bekannten Reaktionen hohe Reinheit des Edukts in der erfindungsgemäßen Ringschlußreaktion kann eine besonders hohe Reinheit des gewünschten Lamotrigins bis hin zu einer für Arzneimittel notwendigen Reinheit ohne die Notwendigkeit aufwendiger Reinigungsschritte erzielt werden. Insbesondere wurden die in der EP-A-0 963 980 genannten Verunreinigungen der Formeln VII und VIII in nach dem erfindungsgemäßen Verfahren hergestelltem Lamotrigin nicht nachgewiesen.

Die Ringschlußreaktion kann unter milden Bedingungen durch Erwärmen, beispielsweise auf eine Temperatur im Bereich von 100°-170°C, vorzugsweise 130°-170°C durchgeführt werden. Bevorzugt wird die Ringschlußreaktion in Lösung durchgeführt. Als Lösungsmittel für die Verbindung der Formel XII eignen sich alle organischen Lösungsmittel, die keinen negativen Einfluß auf die Reaktion haben, bevorzugt Dimethylsulfoxid oder Dimethylformamid. Besonders bevorzugt sind solche Lösungsmittel, die in dem gewünschten Temperaturbereich sieden, wobei die Reaktion dann bei der Siedetemperatur des Lösungsmittels unter Rückfluß stattfinden kann. Zur Einstellung einer gewünschten Siedetemperatur können auch Lösungsmittelgemische, beispielsweise aus Dimethylsulfoxid oder Dimethylformamid mit Benzol, Toluol oder Xylol eingesetzt werden. Vorzugsweise wird Dimethylformamid als Lösungsmittel verwendet.

Die Ringschlußreaktion sollte unter Ausschluß von Wasser oder zumindest im wesentlichen unter Ausschluß von Wasser durchgeführt werden.

Die Ringschlußreaktion kann mit der freien Base der Verbindung der Formel XII oder mit einem Säureadditionssalz dieser Verbindung durchgeführt werden. Als Säureadditionssalz wird das Dihydrochlorid bevorzugt. Wenn die Ringschlußreaktion mit einem Säureadditionssalz durchgeführt wird, erhält man als Produkt das Säureadditionssalz des Lamotrigins. In diesem Fall kann, wenn gewünscht, die freie Lamotriginbase in einer dem Fachmann bekannten Weise, beispielsweise mit wäßriger Natronlauge in Dimethylformamid, in fast quantitativer Ausbeute erhalten werden.

Die für die Ringschlußreaktion benötigte Reaktionsdauer hängt von den Verfahrensbedingungen und insbesondere der Temperatur, bei der die Reaktion durchgeführt wird, ab. Sie kann beispielsweise im Bereich von 1-24 Stunden liegen. Eine optimale Zeitdauer kann vom Fachmann leicht bestimmt werden.

In einer bevorzugten Ausführungsform des erfindungsgemäßen Verfahrens wird die für die Ringschlußreaktion benötigte Verbindung der Formel XII durch Umsetzung einer Verbindung der Formel XI 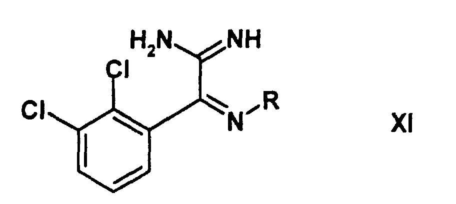 wobei R für einen gegebenenfalls substituierten geradkettigen, verzweigten oder cyclischen Alkyl-, Aryl- oder Aralkyl-Rest steht, oder einem Salz davon mit Aminoguanidin oder einem Salz davon erhalten.

In der Verbindung der Formel XI kann R für einen geradkettigen, verzweigten oder cyclischen Alkylrest, vorzugsweise C₁₋₂₀-, insbesondere C₁₋₁₀-Alkylrest stehen, wie beispielsweise Methyl, Ethyl, n-Propyl, iso-Propyl, n-Butyl, iso-Butyl, tert-Butyl oder Cyclohexyl. Als Arylreste werden für R Phenyl und Naphthyl, insbesondere Phenyl bevorzugt. Als Aralkylreste werden für R Aryl-C₁₋₅-Alkylreste, worin Aryl wie vorstehend definiert ist, und insbesondere Benzyl bevorzugt.

Die vorstehend genannten Alkyl-, Aryl- und Aralkyl- Reste können gegebenenfalls einen oder mehrere, insbesondere 1 oder 2 Substituenten wie beispielsweise Halogen, Hydroxy, C₁₋₆-Alkoxy oder Nitro tragen. Die Aryl- und Aralkyl-Reste können zusätzlich oder alternativ mit C₁₋₆-Alkyl substituiert sein.

Vorzugsweise steht R für Phenyl.

Zur Herstellung der Verbindung der Formel XII wird die Verbindung der Formel XI mit Aminoguanidin oder einem Salz davon, vorzugsweise mit Aminoguanidin-Hydrochlorid, umgesetzt. Die Reaktion findet vorzugsweise in Gegenwart einer starken Mineralsäure, beispielsweise Salzsäure, statt. Als Lösungsmittel eignet sich im Prinzip jedes Lösungsmittel, das sich nicht nachteilig auf die Reaktion auswirkt. Bevorzugt wird die Reaktion in Wasser oder einem Alkohol durchgeführt. Die Reaktionstemperatur ist nicht weiter eingeschränkt und kann beispielsweise in einem Bereich von 40°-120°C liegen.

Wenn die Umsetzung der Verbindung der Formel XI in Anwesenheit von Salzsäure durchgeführt wird, wird das Dihydrochlorid der Verbindung der Formel XII erhalten. Dieses kann wie vorstehend beschrieben entweder unmittelbar durch Ringschlußreaktion zu Lamotrigin-Hydrochlorid umgesetzt werden oder, wenn gewünscht, kann zuvor die frei Base der Verbindung der Formel XII in für den Fachmann bekannter Weise beispielsweise mit wäßriger Natronlauge erhalten werden.

In einer besonders bevorzugten Ausführungsform des erfindungsgemäßen Verfahrens wird die Verbindung der Formel XI oder ein Salz davon durch Umsetzung einer Verbindung der Formel X 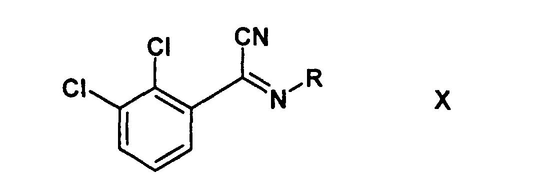 worin R wie vorstehend definiert ist, zunächst mit einer alkoholischen Lösung einer Halogenwasserstoffsäure und anschließend mit NH₃ erhalten. Dieser Reaktionsweg ist deshalb besonders vorteilhaft, da die Verbindung der Formel X überraschend stabil und durch Kristallisation gut zu reinigen ist. Im Gegensatz zu dem im Stand der Technik bei der Synthese von Lamotrigin eingesetzten, zu der Verbindung der Formel X analogen 2,3-Dichlorbenzoylcyanid, das als öliges, instabiles Produkt nur schlecht zu reinigen ist und das daher wesentlich zur Verunreinigung des am Ende der Synthese erhaltenen Lamotrigins beiträgt, ist die Verbindung der Formel X kristallin und stabil, so daß sie leicht gereinigt werden kann. Daher ermöglicht das erfindungsgemäße Verfahren schon an dieser Stelle des Synthesewegs die Herstellung eines besonders reinen Lamotrigins, da bereits die Zwischenstufen in besonders reiner Form erhalten werden können. Darüber hinaus kann die Verbindung der Formel X, wie nachfolgend noch erläutert werden wird, durch Umsetzung mit Natriumcyanid an Stelle von dem im Stand der Technik für die Synthese von 2,3-Dichlorbenzoylcyanid benötigten Kupfercyanid erhalten werden. Da Natriumcyanid im Vergleich zu Kupfercyanid billiger ist, kann die Synthese des Lamotrigins mittels des erfindungsgemäßen Verfahrens damit insgesamt besonders kostengünstig und wirtschaftlich durchgeführt werden.

Die Umsetzung der Verbindung der Formel X zu der Verbindung der Formel XI erfolgt in zwei Stufen, wobei zunächst mit einer alkoholischen Lösung einer Halogenwasserstoffsäure, vorzugsweise einer methanolischen oder ethanolischen Salzsäurelösung, und anschließend mit NH₃, beispielsweise durch Zugabe von mit Ammoniak gesättigtem Ethanol, umgesetzt wird. Beide Reaktionsschritte können beispielsweise in einem Temperaturbereich von -30° bis +10°C durchgeführt werden.

Wenn die Reaktion der Verbindung der Formel X in Gegenwart von Salzsäure durchgeführt wird, erhält man das Hydrochlorid der Verbindung der Formel XI. Dieses kann gegebenenfalls nach Reinigung, beispielsweise durch Umkristallisation, ohne Freisetzung der freien Base in dem erfindungsgemäßen Verfahren weiter eingesetzt werden.

Die α-Iminonitrile der Formel X sind beispielsweise wie in der deutschen Offenlegungsschrift Nr. 2 221 771 beschrieben leicht zugänglich. In einer vorliegend bevorzugten Ausführungsform des erfindungsgemäßen Verfahrens wird jedoch zur Herstellung der Verbindung der Formel X ein Nitron der Formel IX 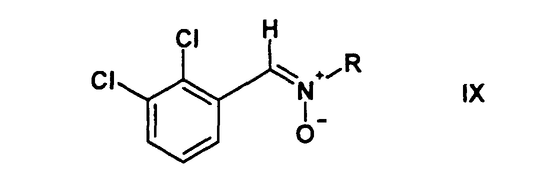 worin R wie vorstehend definiert ist, mit einem Cyanid umgesetzt. Diese Verfahrensvariante hat gegenüber den im Stand der Technik bekannten Verfahren zur Herstellung von Lamotrigin den Vorteil, daß als Cyanid beispielsweise Natriumcyanid eingesetzt werden kann, welches gegenüber dem in den Verfahren des Standes der Technik eingesetzten Kupfercyanid preiswerter und einfacher zu handhaben ist.

Die Umsetzung des Nitrons der Formel IX mit dem Cyanid, vorzugsweise Natriumcyanid, kann beispielsweise in einem wäßrigen Puffer, z.B. einem Phosphat-, Acetat- oder Tartrat-Puffer, insbesondere einem Phosphat-Puffer, beispielsweise bei einem pH-Wert im Bereich von 4-8 durchgeführt werden. Vorteilhaft wird als Co-Solvens ein niedriger Alkohol, vorzugsweise Methanol, eingesetzt. Die Umsetzung kann in einem weiten Temperaturbereich beispielsweise von Raumtemperatur bis etwa 80°C erfolgen.

Das Nitron der Formel IX kann beispielsweise nach dem von M.P. Grammaticakis in Bull. Soc. Chim. Fr. [1951], S. 971 beschriebenen Verfahren erhalten werden.

Die vorliegende Erfindung betrifft darüber hinaus Verbindungen der Formeln IX, X, XI und XII sowie deren Salze, die als Zwischenprodukte in dem erfindungsgemäßen Verfahren auftreten. Darüber hinaus betrifft die vorliegende Erfindung die Verwendung von Verbindungen der Formeln IX, X, XI und XII oder Salzen davon zur Herstellung von Lamotrigin.

Das nach dem erfindungsgemäßen Verfahren hergestellte Lamotrigin eignet sich zur Herstellung von pharmazeutischen Zusammensetzungen, wobei insbesondere pharmazeutisch verträgliche Salze des Lamotrigins eingesetzt werden. Geeignete pharmazeutisch verträgliche Salze und geeignete pharmazeutische Zusammensetzungen sind beispielsweise in EP-A-0 021 121, EP-A-0 247 892 und WO 96/20935 beschrieben.

Das erfindungsgemäße Verfahren wird durch die nachfolgenden Beispiele näher erläutert, ohne es jedoch darauf einzuschränken.

### Beispiel 1

### α-(Phenylimino)-2,3-dichlorphenylacetonitril (X)

In 5,6 l einer Mischung (1:1) aus einer auf pH 5 eingestellten wäßrigen 0,5 molaren Lösung von Kaliumdihdrogenphosphat und Methanol werden 635 mMol N-(2,3-Dichlorphenylmethylen)anilin-N-oxid (IX) gelöst. Zu dieser Lösung werden bei ca. 50°C 65 g (1,3 Mol) Natriumcyanid in 200 ml Wasser gegeben. Während der Reaktion wird der pH-Wert zwischen 8,0 und 8,5 konstant gehalten. Nach Beendigung der Umsetzung (DC-Kontrolle) wird die Reaktionsmischung neutralisiert und auf 0°C abgekühlt. Die abgeschiedenen gelbbraunen Kristalle werden in Methylenchlorid gelöst. Die organische Phase wird mit Wasser gewaschen, getrocknet und im Vakuum evaporiert. Der Rückstand wird aus wenig Methanol umkristallisiert.
- Ausbeute:: 157 g
- Reinheit:: 99,0% (HPLC)
- Schmelzpunkt:: 65°-67°C

Die NMR-spektroskopischen Daten entsprechen der chemischen Struktur.

### Beispiel 2

### α-(Phenylimino)-2,3-dichlorphenylacetamidin-Hydrochlorid (XI·HCl)

In eine ethanolische Salzsäurelösung (hergestellt aus 400 ml Ethanol, 240 ml Thionylchlorid und 52 ml Wasser) werden 151,2 g α-(Phenylimino)-2,3-dichlorphenylacetonitril (X) bei - 10°C eingetragen und bei dieser Temperatur mehrere Stunden gerührt. Anschließend wird das Reaktionsgemisch bei -10°C zu 6,5 l Ethanol, das mit Ammoniak gesättigt ist, gegeben und 12 h bei Raumtemperatur gerührt. Nach dem Einengen der Reaktionslösung wird das Produkt durch Zugabe von Wasser ausgefällt, abfiltriert und getrocknet. Das Produkt (XI•HCl) kann zur weiteren Reinigung aus Aceton umkristallisiert werden.
- Ausbeute:: 166,7 g
- Reinheit:: 99,7% (HPLC)
- Schmelzpunkt:: 263°-267°C

Die NMR-spektroskopischen Daten entsprechen der chemischen Struktur.

### Beispiel 3

### α-Oxo-2,3-dichlorphenylacetamidino-aminoguanidino-hydrazon-Dihydrochlorid (XII•2HCl)

In eine Lösung von 83,2 g (550 mMol) Aminoguanidin-Bicarbonat (Reinheit: 90%) in 500 ml 10 m Salzsäure werden nach dem Abklingen der Gas-Entwicklung 82,5 g (250 mMol) α-Phenylimino-2,3-dichlorphenylacetamidin-Hydrochlorid (XI•HCl) eingetragen und zum Rückfluß erhitzt. Nach einer weiteren Zugabe der gleichen Menge α-Phenylimino-2,3-dichlorphenylacetamidin-Hydrochlorid (XI•HCl) wird das Reaktionsgemisch bis zur vollständigen Umsetzung mehrere Stunden weiter auf dieser Temperatur gehalten.

Nach Beendigung der Reaktion wird auf 0°C abgekühlt und das ausgefallene Produkt wird abfiltriert und mit Eiswasser gewaschen. Durch längeres Halten im Vakuum bei leicht erhöhter Temperatur kann das hygroskopische Produkt weitgehend getrocknet werden.
- Ausbeute:: 160,0 g (91,5%)
- Reinheit:: 99,0% (HPLC)
- Schmelzpunkt:: 218°-220°C (Zers.)

### Beispiel 4

### α-Oxo-2,3-dichlorphenylacetamidino-aminoguanidino-hydrazon (XII)

Die freie Base des Dihydrochlorids (XII•2HCl) kann in der üblichen Weise mit wäßriger Natronlauge erhalten werden.
- Schmelzpunkt:: 200°-203°C

### Beispiel 5

### Lamotrigin-Hydrochlorid (I•HCl)

20,76 g α-Oxo-2,3-dichlorphenylacetamidino-aminoguanidino-hydrazon-Dihydrochlorid (XII•2HCl) (Wassergehalt unter 0,05 %) werden in trockenem Dimethylformamid ca. 20 Stunden unter Rückfluß erhitzt. Der nach dem Abziehen des Lösungsmittels erhaltene Rückstand wird kurz mit 200 ml Isopropanol zum Sieden erhitzt und danach auf 20°C abgekühlt. Die ausgeschiedenen Kristalle werden abfiltriert und aus wäßrigem Isopropanol (Volumenverhältnis 1:1) umkristallisiert und im Vakuum bei 80°C getrocknet.
- Ausbeute:: 10,6 g (60,5 %)
- Reinheit:: 99,5 % (HPLC)
- Schmelzpunkt:: 233°-235°C (Zers.)

Die freie Lamotrigin-Base kann in der üblichen Weise durch Freisetzung mit wäßriger Natronlauge in Dimethylformamid in fast quantitativer Ausbeute erhalten werden.

Schmelzpunkt: 216°-217°C

### Beispiel 6

### Lamotrigin (1)

8,19 g α-Oxo-2,3-dichlorphenylacetamidino-aminoguanidino-hydrazon (XII) (Wassergehalt max. 0,3 %) werden in 150 ml trockenem Dimethylformamid bei 60°C gelöst. Nach der Einengung der Lösung unter Vakuum um rund 20% wird die Temperatur auf Rückflußtemperatur erhöht und 1,5 h bei dieser Temperatur belassen. Nach dem Einengen des Reaktionsgemisches und Abkühlung auf Raumtemperatur können 6,7 g Rohprodukt isoliert werden, das zur Reinigung aus Isopropanol umkristallisiert wird.
- Ausbeute:: 5,1 g (66,7 %)
- Reinheit:: 99,9 % (HPLC)
- Schmelzpunkt:: 215,5°-216,5°C (Zers.)

## Patentansprüche

1. Verfahren zur Herstellung einer Verbindung der Formel I 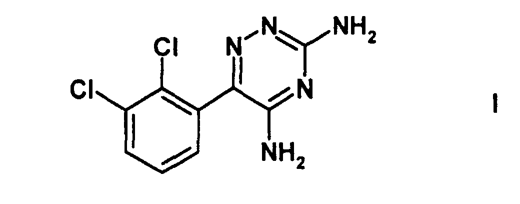 oder eines pharmazeutisch verträglichen Salzes davon, worin mit einer Verbindung der Formel XII 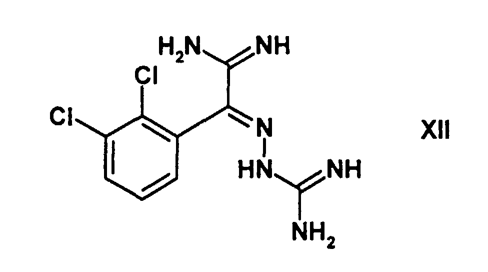 oder einem Salz davon eine Ringschlußreaktion durchgeführt wird und gegebenenfalls aus dem dabei erhaltenen Salz der Verbindung der Formel I die freie Base freigesetzt wird und, falls gewünscht, diese in ein pharmazeutisch verträgliches Salz überführt wird.

2. Verfahren nach Anspruch 1, worin die Ringschlußreaktion durch Erwärmen durchgeführt wird.

3. Verfahren nach Anspruch 2, worin die Ringschlußreaktion bei einer Temperatur im Bereich von 100° bis 170°C durchgeführt wird.

4. Verfahren nach einem der vorhergehenden Ansprüche, worin die Ringschlußreaktion mit einem Säureadditionssalz, vorzugsweise dem Dihydrochlorid der Verbindung der Formel XII durchgeführt wird.

5. Verfahren nach einem der vorhergehenden Ansprüche, worin die Verbindung der Formel XII oder ein Salz davon durch Umsetzung einer Verbindung der Formel XI 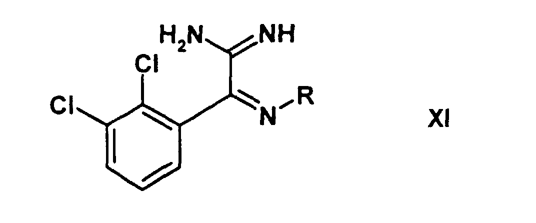 wobei R für einen gegebenenfalls substituierten geradkettigen, verzweigten oder cyclischen Alkyl-, Aryl- oder Aralkyl-Rest steht, oder einem Salz davon mit Aminoguanidin oder einem Salz davon erhalten wird.

6. Verfahren nach Anspruch 5, worin die Verbindung der Formel XI oder ein Salz davon durch Umsetzung einer Verbindung der Formel X 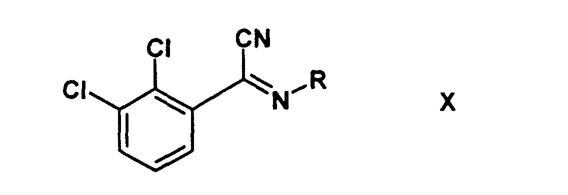 wobei R wie in Anspruch 5 definiert ist, zunächst mit einer alkoholischen Lösung einer Halogenwasserstoffsäure und anschließend mit NH₃ erhalten wird.

7. Verfahren nach Anspruch 6, worin die Verbindung der Formel X oder ein Salz davon durch Umsetzung einer Verbindung der Formel IX 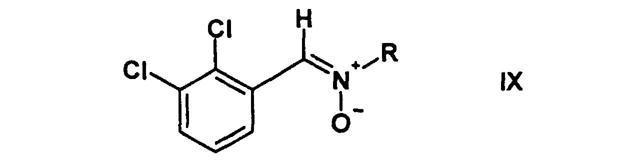 wobei R wie in Anspruch 5 definiert ist, mit einem Cyanid erhalten wird.

8. Verbindung der Formel IX 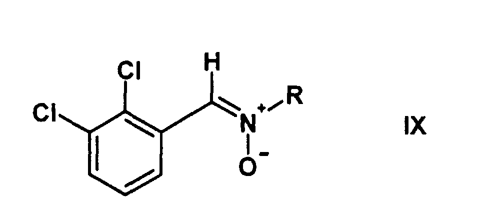 wobei R für einen gegebenenfalls substituierten geradkettigen, verzweigten oder cyclischen Alkyl-, Aryl- oder Aralkyl-Rest steht, oder ein Salz davon.

9. Verbindung der Formel X 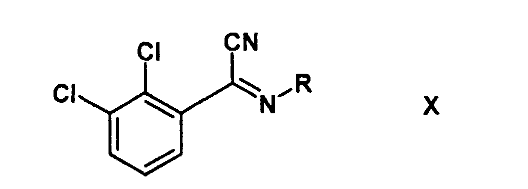 wobei R für einen gegebenenfalls substituierten geradkettigen, verzweigten oder cyclischen Alkyl-, Aryl- oder Aralkyl-Rest steht, oder ein Salz davon.

10. Verbindung der Formel XI 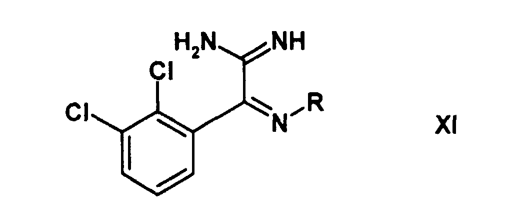 wobei R für einen gegebenenfalls substituierten geradkettigen, verzweigten oder cyclischen Alkyl-, Aryl- oder Aralkyl-Rest steht, oder ein Salz davon.

11. Verbindung der Formel XII 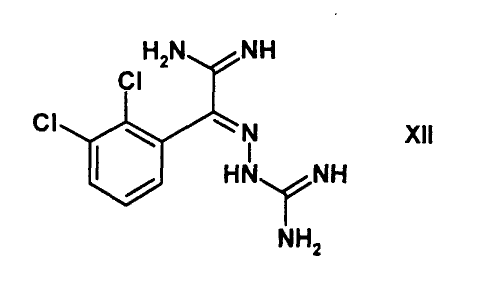 oder ein Salz davon.

12. Verwendung einer Verbindung der Formel IX 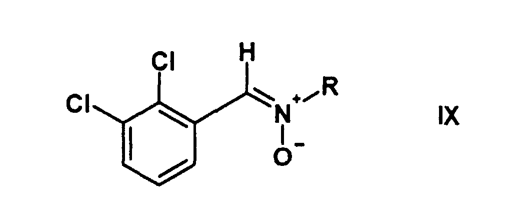 wobei R für einen gegebenenfalls substituierten geradkettigen, verzweigten oder cyclischen Alkyl-, Aryl- oder Aralkyl-Rest steht, oder einem Salz davon zur Herstellung von Lamotrigin oder einem pharmazeutisch verträglichen Salz davon.

13. Verwendung einer Verbindung der Formel X 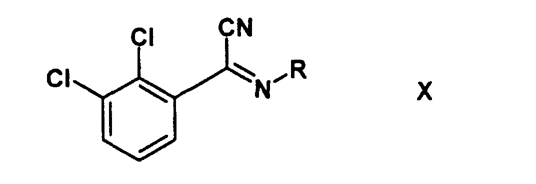 wobei R für einen gegebenenfalls substituierten geradkettigen, verzweigten oder cyclischen Alkyl-, Aryl- oder Aralkyl-Rest steht, oder einem Salz davon zur Herstellung von Lamotrigin oder einem pharmazeutisch verträglichen Salz davon.

14. Verwendung einer Verbindung der Formel XI 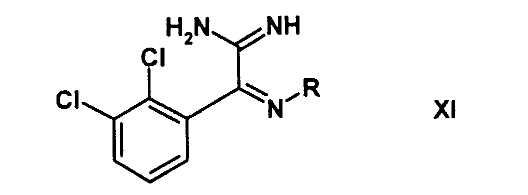 wobei R für einen gegebenenfalls substituierten geradkettigen, verzweigten oder cyclischen Alkyl-, Aryl- oder Aralkyl-Rest steht, oder einem Salz davon zur Herstellung von Lamotrigin oder einem pharmazeutisch verträglichen Salz davon.

15. Verwendung einer Verbindung der Formel XII 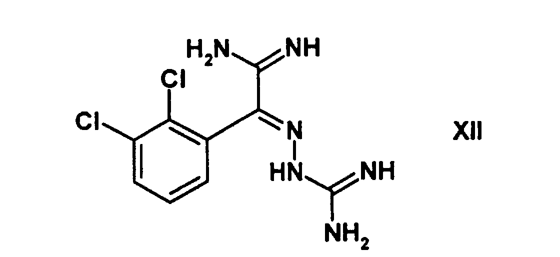 oder einem Salz davon zur Herstellung von Lamotrigin oder einem pharmazeutisch verträglichen Salz davon.

## Claims

1. Method for producing a compound of formula I 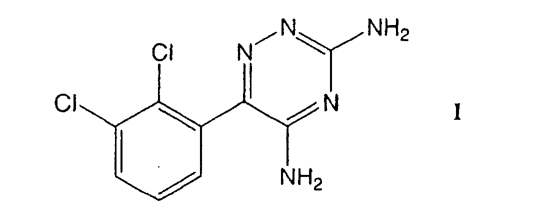 or a pharmaceutically acceptable salt thereof, in which a cyclization reaction is performed with a compound of formula XII 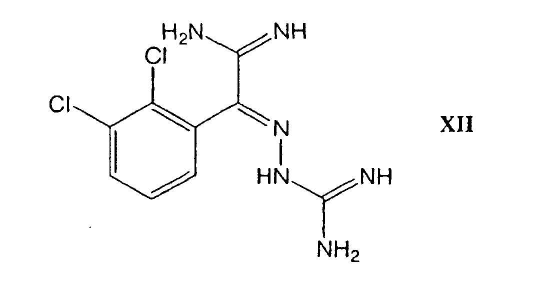 or a salt thereof and, optionally, from which salt of the compound of formula I thus obtained the free base is released and, if desired, said free base is converted to a pharmaceutically acceptable salt.

2. Method according to claim 1, in which the cyclization reaction is performed by heating.

3. Method according to claim 2, in which the cyclization reaction is performed at a temperature in the range of between 100°C and 170°C.

4. Method according to one of the preceding claims, in which the cyclization reaction is performed using an acid addition salt, preferably the dihydrochloride of the compound of formula XII.

5. Method according to one of the preceding claims, in which the compound of formula XII or a salt thereof is obtained by reacting a compound of formula XI 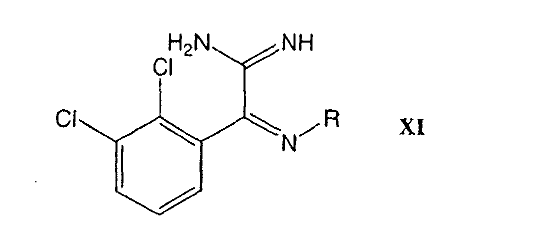 in which R is an optionally substituted straight- or branched-chained, or cyclical alkyl-, aryl- or aralkyl-residue, or a salt thereof, with aminoguanidine or a salt thereof.

6. Method according to claim 5, in which the compound of formula XI or a salt thereof is obtained by reacting a compound of formula X 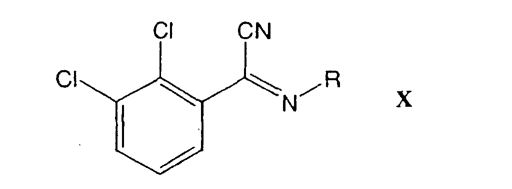 in which R is defined as in claim 5, first with. an alcohol solution of a hydrohalic acid, then with NH₃.

7. Method according to claim 6, in which the compound of formula X or a salt thereof is obtained by reacting a compound of formula IX 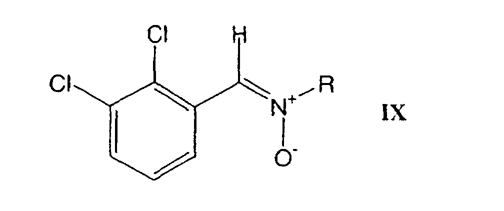 in which R is defined as in claim 5, with a cyanide.

8. Compound of formula IX 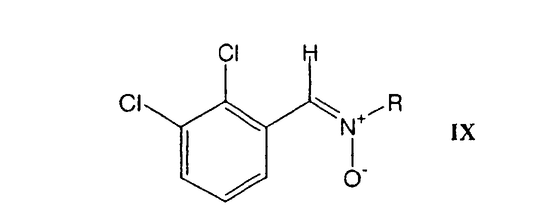 in which R is an optionally substituted straight- or branched-chained, or cyclical alkyl-, aryl-, or aralkyl-residue, or a salt thereof.

9. Compound of formula X 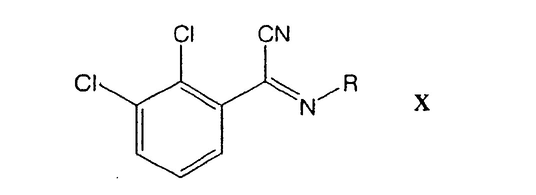 in which R is an optionally substituted straight- or branched-chained, or cyclical alkyl-, aryl-, or aralkyl-residue, or a salt thereof.

10. Compound of formula XI 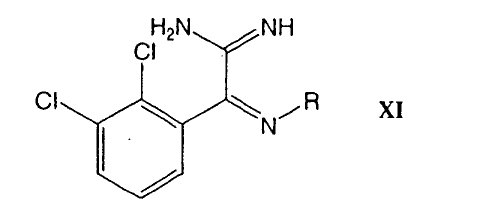 in which R is an optionally substituted straight- or branched-chained, or cyclical alkyl-, aryl-, or aralkyl-residue, or a salt thereof.

11. Compound of formula XII 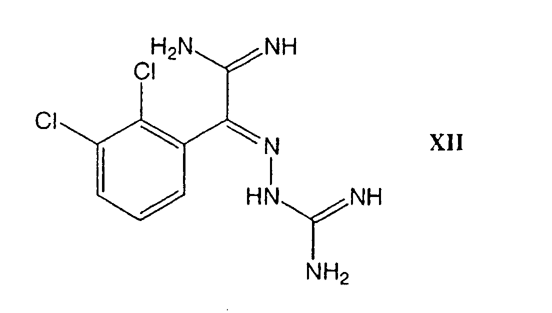 or a salt thereof.

12. Use of a compound of formula IX 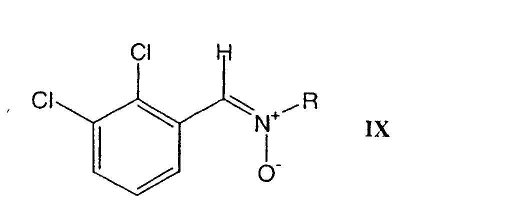 in which R is an optionally substituted straight- or branched-chained, or cyclical alkyl-, aryl-, or aralkyl-residue, or a salt thereof for producing lamotrigine or a pharmaceutically acceptable salt thereof.

13. Use of a compound of formula X 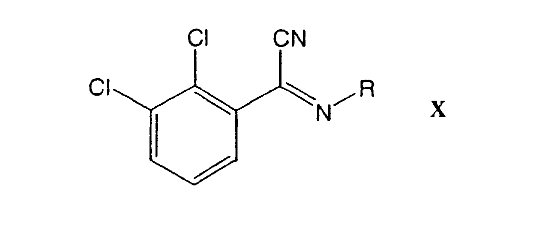 in which R is an optionally substituted straight- or branched-chained, or cyclical alkyl-, aryl-, or aralkyl-residue, or a salt thereof for producing lamotrigine or a pharmaceutically acceptable salt thereof.

14. Use of a compound of formula XI 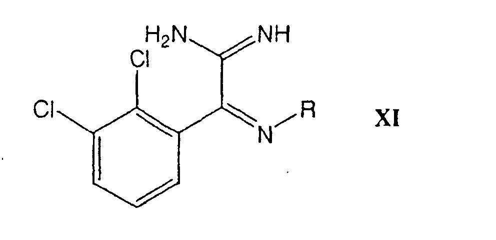 in which R is an optionally substituted straight- or branched-chained, or cyclical alkyl-, aryl-, or aralkyl-residue, or a salt thereof for producing lamotrigine or a pharmaceutically acceptable salt thereof.

15. Use of a compound of formula XII 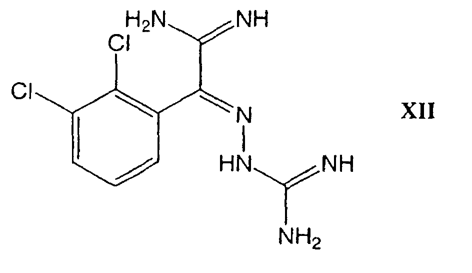 or a salt thereof for producing lamotrigine or a pharmaceutically acceptable salt thereof.

## Revendications

1. Procédé de préparation d'un composé de formule I 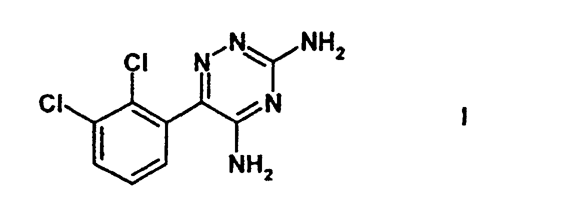 ou d'un sel de celui-ci acceptable sur le plan pharmaceutique, dans lequel on réalise une réaction de cyclisation avec un composé de formule XII 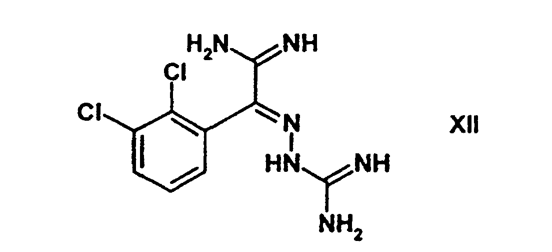 ou avec un sel de celui-ci, et on libère éventuellement la base libre du sel du composé de formule I ainsi obtenu et, si souhaité, on la convertit en un sel acceptable sur le plan pharmaceutique.

2. Procédé selon la revendication 1, dans lequel on réalise la réaction de cyclisation au moyen d'un chauffage.

3. Procédé selon la revendication 2, dans lequel on réalise la réaction de cyclisation à une température allant de 100°C à 170°C.

4. Procédé selon l'une quelconque des revendications précédentes, dans lequel on réalise la réaction de cyclisation avec un sel d'addition d'acide, de préférence avec le dichlorhydrate du composé de formule XII.

5. Procédé selon l'une quelconque des revendications précédentes, dans lequel on obtient le composé de formule XII ou un sel de celui-ci en faisant réagir un composé de formule XI 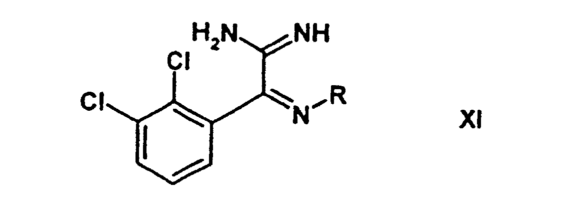 dans laquelle R représente un groupe alkyle, aryle ou aralkyle cyclique, linéaire ou ramifié, éventuellement substitué, ou un sel de celui-ci avec de l'aminoguanidine ou un sel de celle-ci.

6. Procédé selon la revendication 5, dans lequel on obtient le composé de formule XI ou un sel de celui-ci en faisant réagir un composé de formule X 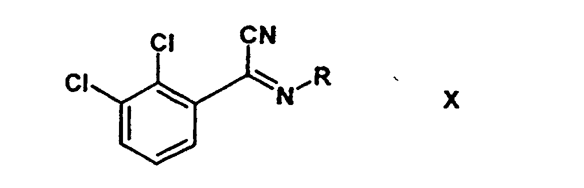 dans laquelle R est tel que défini dans la revendication 5, d'abord avec une solution alcoolique d'un acide halogénohydrique puis avec NH₃.

7. Procédé selon la revendication 6, dans lequel on obtient le composé de formule X ou un sel de celui-ci en faisant réagir un composé de formule IX 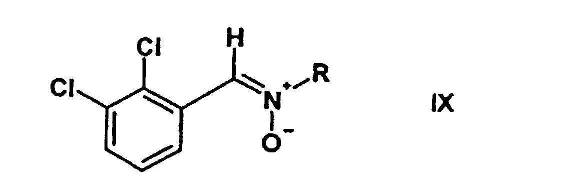 dans laquelle R est tel que défini dans la revendication 5, avec un cyanure.

8. Composé de formule IX 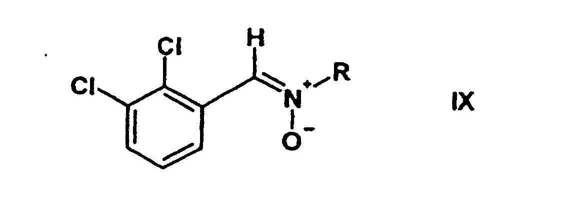 dans laquelle R représente un groupe alkyle, aryle ou aralkyle cyclique, linéaire ou ramifié, éventuellement substitué, ou un sel de celui-ci.

9. Composé de formule X 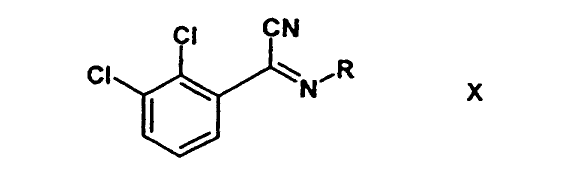 dans laquelle R représente un groupe alkyle, aryle ou aralkyle cyclique, linéaire ou ramifié, éventuellement substitué, ou un sel de celui-ci.

10. Composé de formule XI 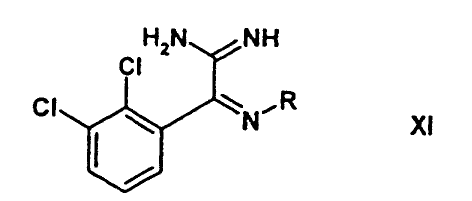 dans laquelle R représente un groupe alkyle, aryle ou aralkyle cyclique, linéaire ou ramifié, éventuellement substitué, ou un sel de celui-ci.

11. Composé de formule XII 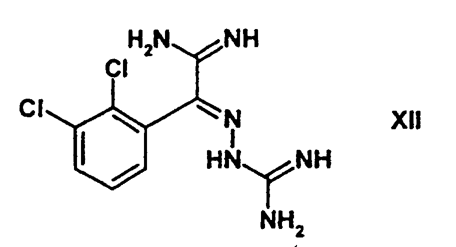 ou un sel de celui-ci.

12. Utilisation d'un composé de formule IX 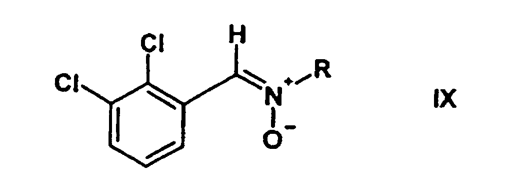 dans laquelle R représente un groupe alkyle, aryle ou aralkyle cyclique, linéaire ou ramifié, éventuellement substitué, ou un sel de celui-ci pour la préparation de lamotrigine ou d'un sel de celle-ci acceptable sur le plan pharmaceutique.

13. Utilisation d'un composé de formule X 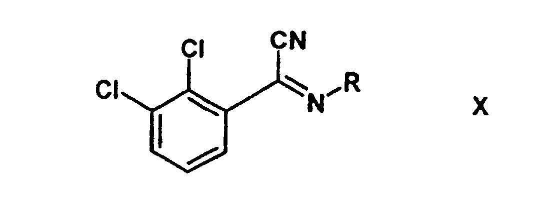 dans laquelle R représente un groupe alkyle, aryle ou aralkyle cyclique, linéaire ou ramifié, éventuellement substitué, ou un sel de celui-ci pour la préparation de lamotrigine ou d'un sel de celle-ci acceptable sur le plan pharmaceutique.

14. Utilisation d'un composé de formule XI 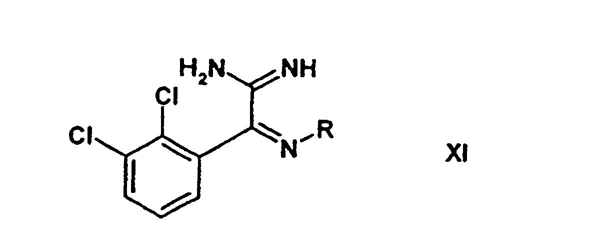 dans laquelle R représente un groupe alkyle, aryle ou aralkyle cyclique, linéaire ou ramifié, éventuellement substitué, ou un sel de celui-ci pour la préparation de lamotrigine ou d'un sel de celle-ci acceptable sur le plan pharmaceutique.

15. Utilisation d'un composé de formule XII 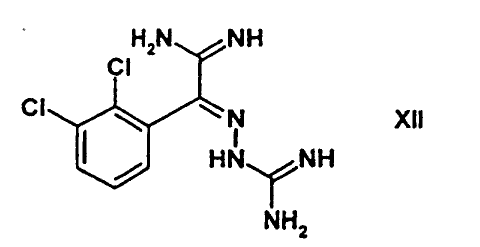 ou d'un sel de celui-ci pour la préparation de lamotrigine ou d'un sel de celle-ci acceptable sur le plan pharmaceutique.
